# EUROPÄISCHE PATENTSCHRIFT

(11) **EP 1 499 294 B1**
(45) Veröffentlichungstag und Bekanntmachung des Hinweises auf die Patenterteilung: **11.08.2010**
(21) Anmeldenummer: 03727314.1
(22) Anmeldetag: 16.04.2003
(51) Int. Cl.: A61K 9/127, A61K 9/16, A61K 31/355, A61K 31/015

(54) **POLYMERISIERTE PEPTID-MODIFIZIERTE LIPIDE ALS BIOLOGISCHE TRANSPORTSYSTEME FÜR MIKRONUTRIENTS**
POLYMERIZED LIPIDS MODIFIED WITH PEPTIDES USED AS DELIVERY SYSTEM FOR MICRONUTRIENTS
SYSTEME DE TRANSPORT POUR DES MICRONUTRIENTS COMPRENANT DES LIPIDES POLYMERISES MODIFIES AVEC DES PEPTIDES

(30) Priorität: 29.04.2002 AT 6562002
(43) Veröffentlichungstag der Anmeldung: 26.01.2005
(73) Patentinhaber: BIOTESYS GMBH, 73728 Esslingen (DE)
(72) Erfinder: BERNHARDT, Jürgen, Wilhelm, Richard, 71384 Weinstadt (DE); HEINRICH, Felix, Oliver, 73733 Esslingen (DE); ENGELHART-JENTZSCH, Karin, 67063 Ludwigshafen (DE)
(74) Vertreter: Secklehner, Günter
(86) Internationale Anmeldenummer: PCT/EP2003/003973
(87) Internationale Veröffentlichungsnummer: WO 2003/092647

(56) Entgegenhaltungen:
- EP-A- 0 497 997
- WO-A-01/60848
- WO-A-99/33493
- LESTINI B J ET AL: "Surface modification of liposomes for selective cell targeting in cardiovascular drug delivery" JOURNAL OF CONTROLLED RELEASE, ELSEVIER SCIENCE PUBLISHERS B.V. AMSTERDAM, NL, Bd. 78, Nr. 1-3, 17. Januar 2002 (2002-01-17), Seiten 235-247, XP004329821 ISSN: 0168-3659
- DATABASE SWALL [Online] EBI; 1. Oktober 1996 (1996-10-01) "CAD4_Human" retrieved from WWW.EBI.AC.UK Database accession no. P55283 XP002255166

### Sequenzprotokoll

<110> BioTeSys GmbH
   Schelztorstrasse 54-56
   D 73728 Esslingen
   DEUTSCHLAND
<120> Transportsystem in biologischen Systemen
<150> A 656/2002
   <151> 2002-04-29
<160> 15
<210> 1
   <211>6
   <212> PRT
   <213> Künstliche Sequenz
<220>
   <223> Oligopeptid
<400> 1
   Gly Arg Gly Asp Ser Pro
   1 5
<210> 2
   <211> 5
   <212> PRT
   <213> Künstliche Sequenz
<220>
   <223> Oligopeptid
<400>2
   Tyr Ile Glu Ser Arg
   1 5
<210> 3
   <211>5
   <212> PRT
   <213> Künstliche Sequenz
<220>
   <223> Oligopeptid
<400> 3
   Ala Asp Gly Glu Ala
   1 5
<210> 4
   <211> 6
   <212> PRT
   <213> Künstliche Sequenz
<220>
   <223> Oligopeptid
<400> 4
   Val Arg Leu Leu Asn Asn
   1 5
<210> 5
   <211> 8
   <212> PRT
   <213> Künstliche Sequenz
<220>
   <223> Oligopeptid
<400> 5
   Val Arg Leu Leu Asn Asn Trp Asp
   1 5
<210> 6
   <211>8

   <212> PRT
   <213> Künstliche Sequenz
<220>
   <223> Oligopeptid
<400> 6
   Gly Arg Val Arg Leu Leu Asn Asn
   1 5
<210> 7
   <211> 6
   <212> PRT
   <213> Künstliche Sequenz
<220>
   <223> Oligopeptid
<400> 7
   Met Thr Ala Gly Ala Gly
   1 5
<210> 8
   <211> 6
   <212> PRT
   <213> Künstliche Sequenz
<220>
   <223> Oligopeptid
<400> 8
   Leu Ser Gly Ala Leu Arg
   1 5
<210>9
   <211>22
   <212> PRT
   <213> Künstliche Sequenz
<220>
   <223> Oligopeptid
<400> 9
   Ile Val Ala Ile Leu Ile Cys Ile Leu Ile Leu Leu Thr Met Val Leu Leu Phe Val Met Trp Met
   1 5 10 15 20
<210> 10
   <211> 12
   <212> PRT
   <213> Künstliche Sequenz
<220>
   <223> Oligopeptid
<400> 10
   Ile Val Ala Ile Leu Ile Cys Ile Leu Ile Leu Leu
   1 5 10
<210> 11
   <211> 18
   <212> PRT
   <213> Künstliche Sequenz
<220>
   <223> Oligopeptid
<400> 11
   Ile Val Ala Ile Leu Ile Cys Ile Leu Ile Leu Leu Thr Met Val Leu Leu Phe
   1 5 10 15
<210> 12

   <211>6
   <212> PRT
   <213> Künstliche Sequenz
<220>
   <223> Oligopeptid
<400> 12
   Ile Val Ala Ile Leu Ile
   1 5
<210> 13
   <211> 6
   <212> PRT
   <213> Künstliche Sequenz
<220>
   <223> Oligopeptid
<400> 13
   Cys Ile Leu Ile Leu Leu
   1 5
<210> 14
   <211> 6
   <212> PRT
   <213> Künstliche Sequenz
<220>
   <223> Oligopeptid
<400> 14
   Thr Met Val Leu Leu Phe
   1 5
<210> 15
   <211> 6
   <212> PRT
   <213> Künstliche Sequenz
<220>
   <223> Oligopeptid
<400> 15
   Leu Phe Val Met Trp Met
   1 5

## Beschreibung

Die Erfindung betrifft ein Transportsystem für Wirkstoffe umfassend Hybridpartikel aus zumindest einer Schicht von Lipidmolekülen und zumindest einen über eine Spacereinheit verbundenen Liganden, welcher ein Peptid ist, dadurch gekennzeichnet, daß zumindest eine polymerisierbare Gruppe in die Hybridpartikel eingebaut ist.

Weiters betrifft die Erfindung ein Verfahren zum Transport von Wirkstoffen.

Die Erfindung betrifft außerdem auch ein Transportsystem zur Verwendung als Arzneimittel.

Des weiteren betrifft die Erfindung auch die Verwendung des Transportsystems zur Herstellung eines Arzneimittels zur Behandlung von Ernährungsdefiziten.

Heutzutage ist bekannt, dass eine Vielzahl von Krankheiten, wie z.B. chronische oder akute inflammatorische Prozesse, Infekte, Ischämie, etc., durch allgemein defizitäre oder pathophysiologische Zustände, die durch einen systemischen oder lokalen Mangel an Mikronährstoffen gekennzeichnet sind, verursacht werden. Die Zufuhr von Mikronutrienten ist für normale zelluläre Funktionen und damit für die Aufrechterhaltung bzw. Regeneration von Zellverbänden eine wichtige Voraussetzung. Eine zentrale Bedeutung unter den Mikronutrienten kommt dabei auch den fettlöslichen Vitaminen für den reibungslosen Ablauf der allgemeinen physiologischen Prozesse zu. Ihre Metabolisierung unterliegt einer sehr effizienten Kontrolle und oftmals auch einer homöostatischen Regulation. Diese physiologische Balancierung kollidiert in gewisser Weise mit Indikationen, bei denen es aus medizinischer, insbesondere aus ernährungsmedizinischer, Sicht angeraten wäre, lokal hohe Mikronutrientenkonzentrationen zu applizieren. Bei solchen Indikationen kann die systemische Balance nur durch die Verabreichung von sehr hohen Mikronutrientenkonzentrationen umgangen werden. Als Folge davon kann es allerdings teilweise zu starken Belastungen des gesamten Organismus, insbesondere der Leber aber auch der Nieren, durch die hohe Mikronutrientenkonzentration kommen.

Eine Transportmöglichkeit ist hydrophile Polymergele natürlichen und synthetischen Ursprungs (sogenannte Hydrogele, z.B. Alginat, Fibrin, Poly-Lactat-Glycolsäurecopolymer (GLGLA) etc.). Der Transport von hydrophoben Wirkstoffen mit Hilfe dieser polymeren Materialien ist jedoch nicht einfach, weil polymere Hydrogele im allgemeinen wasserquellbare Substanzen sind, und das Beladen z.B. mit hydrophoben Mikronährstoffen (wie z.B. fettlöslichen Vitaminen bzw. Vitaminvorstufen) ohne weitere Modifikationen des Hydrogels nicht möglich ist.

Liposome, welche an der Oberfläche kurze wasserquellbare Polymerketten (wie z.B. Polyethylenglykol (PEG)) tragen, verfügen ebenfalls über eine erhöhte Langzeitstabilität. Dies kann über die Entropieelastizität der eingesetzten Polymere an der Oberfläche der Liposomen erklärt werden. Die ersten Moleküle, welche in einer therapeutischen Behandlung als erstes in Kontakt mit den eingesetzten Partikeln treten, sind Proteine. Infolge einer Adsorption dieser Proteine wird eine Signalkaskade ausgelöst, die letztlich die "Entsorgung" der vermeintlichen Fremdkörper durch das Immunsystem veranlasst. Die Adsorption von Proteinen auf der Oberfläche von Liposomen, welche kurze Polymerketten tragen, bewirkt, dass diese Polymere zusammengestaucht werden. Durch die Einschränkung der Konformationsfreiheit in dieser Situation verbraucht das Polymer sehr viel Energie in Form von Entropie. Diesem energetisch ungünstigen Zustand entkommt das Polymer, indem es sich wieder streckt, was zu einer Repulsion des Proteins führt, analog einer Feder, welche gestaucht wird und sich wieder entspannt. Somit unterbindet dieser Prozess eine Adsorption von Proteinen und letztlich eine Reaktion des Immunsystems. Parameter, welche diesen repulsiven Prozess beeinflussen, sind die Oberflächenkonzentration und die Länge (molekulare Masse) der Polymere sowie das Phasenverhalten in Mischungen mit weiteren molekularen Bestandteilen der Lipsome.

Liposomale Systeme in denen PEG-modifizierte Lipide eingebaut werden zeigen in humanen Studien Halbwertszeiten von bis zu 45 Stunden. Viele Wirkstoffadministrationen, in denen Liposome und Polymer-funktionalisierte Liposome zum Einsatz kommen, beruhen auf einer passiven Zuführung der Wirkstoffe in bestimmte Gewebe. Dies bedeutet, dass eine therapeutische Verbesserung letztlich auf den langen Verweilzeiten der Partikel im Körper beruhen. Mit diesen Ansätzen kann man allerdings nicht ausschließen, dass die Wirkstoffe sich letztlich in verschiedensten Geweben gleichzeitig anreichern, auch in solchen, wo der Wirkstoff nicht gebraucht oder im ungünstigsten Fall auch noch unerwünschte Nebeneffekte verursacht.

Liposomale Strukturen sind ein geeignetes Trägersystem für Wirkstoffe und ermöglichen den Transport und die Freisetzung solcher Stoffe. Gerade Transportsysteme, welche auf der Basis von liposomalen Strukturen hergestellt werden, haben in den letzten Jahren stark an therapeutischer Bedeutung gewonnen und werden bereits vielfach kommerziell eingesetzt. Hier macht man sich zu Nutze, dass kleine unilamellare Vesikel (Durchmesser unter 100 nm) nur unzureichend vom menschlichen Immunsystem erkannt und in der Folge nicht von Makrophagen bzw. Monozyten beseitigt werden. Somit erhöht sich die Verweildauer (Halbwertszeiten) dieser Formulierungen signifikant, was therapeutisch von enormen Vorteil ist. Neben Strukturgeometrischer Faktoren, die zu einer erhöhten Langzeitstabilität führt, kann diese auch durch eine entsprechende Einstellung und Kontrolle der physikochemischen Oberflächeneigenschaften (wie z.B. Ladung) der partikulären Systeme erreicht werden. In einem Beispiel wird der Wirkstoff Daunorubicinzitrat in passiven, d.h. "nicht"-funktionalisierten Liposomen verpackt und zur Behandlung von Kaposi Sarkom Läsionen eingesetzt. (Nunez, M., Saballs, P., Valencia, ME., Santos, J., Ferrer, E., Santos, I., Berrocal, A., Galindo, MJ., Podzamczer, D., Gonzlez-Lahoz, J. 2001. Response to liposomal doxorubicin and clinical outcome of HIV-1-infected patients with Kaposi's sarcoma receiving highly active antiretroviral therapy. HIV Clin Trials. 2(5):429-37.) Nachteilig ist, dass die Ziele nicht direkt mit Wirkstoffen versorgt werden können, ohne dabei den gesamten Organismus zu belasten. Dies ist nur durch die Umgehung von Regulationsmechanismen möglich. Lestini et al. (J. Contr. Ref. 78 2002. 235-247) beschreibt eine Oberflächenmodifikation von Liposomer zum Ztal. gerichteten Transport.

Aufgabe der Erfindung ist es eine Möglichkeit zum stabilen zielorientierten Transport von Wirkstoffen in einem biologischen System anzugeben.

Die Aufgabe der Erfindung wird durch das Transportsystem entsprechend den Merkmalen im Kennzeichenteil des Anspruches 1 gelöst. Der Vorteil des Transportsystems liegt darin, dass durch die Kombination des Lipidmoleküls mit einem Peptid das System nicht nur entsprechend lange im Körper verweilt, sondern vielmehr aktiv mit Zellen kommunizieren kann. Von Vorteil ist weiters, dass Transportsysteme, welche über eine definierte Zusammensetzung und Interaktion molekularer Bausteine sowie über kontrollierbare Strukturen im Submikrometer Bereich verfügen, zusätzlich Mikronährstoffe in bestimmten Kompartimenten aufnehmen und zielgerichtet in bestimmte Gewebetypen transportieren können. Solche partikulären Transportsysteme, die neben einer strukturell/funktionalen Definition gleichzeitig aktiv mit biologischen Gewebe kontrollierbar kommunizieren können, sind eine der großen Chance der modernen Biotechnologie. Vorteilhaft ist weiter, dass die Wirkstoffe am Wirkungsort akkumuliert werden können um eine hohe Konzentration am Wirkungsort zu erzielen und nicht eine systemische Belastung des gesamten Organismus hervorgerufen wird.

Von Vorteil ist dabei daß durch das Einfügen einer Spacereinheit zwischen der polaren Kopfgruppe des Lipidmoleküls und dem Peptid sterische Hindernisse und/oder Ladungsinterferenzen bei der Interaktion des Hybridpartikels mit dem Bindungspartner, insbesondere einer Zielzelle, verhindert werden können.

Gemäß Anspruch 2 wird ermöglicht, dass durch die beabstandete Anordnung der Oligopeptide das partikuläre System sowohl an größere, als auch an nicht benachbarte Bindungspartner binden kann, weil es zu keinen Behinderungen durch mangelnde räumliche Ausdehnungsmöglichkeiten kommt.

Gemäß Anspruch 3 erweist sich als vorteilhaft, dass Moleküle verwendet werden können, die vom Immunsystem des Körpers nicht oder nur teilweise erkannt werden und somit nicht zerstört werden. Weiters ist von Vorteil, dass diese Moleküle die Eigenschaft aufweisen, sich selbsttätig zu vesikulären Strukturen zu organisieren.

Vorteilhaft sind weiters Ausgestaltungen nach den Ansprüchen 4 und 5, wonach es durch Rezeptoren, welche als Transmembranproteine in der Zelle verankert sind, zu Wechselwirkungen mit Peptiden, welche Teile der Hybridpartikel des Transportsystems sind und sich außerhalb der Zelle befinden, kommt. Der Einbau von solchen Peptiden in synthetische Biomaterialien definierter Struktur bzw. die Modifikation der Grenzflächen partikulärer synthetischer Transportsysteme ermöglicht die Einflussnahme auf die Wechselwirkung dieser biomimetischen Materialien mit den Zielzellen.

Gemäß der Ausgestaltung im Anspruch 6 können die Wirkstoffe zielgerichtet an den Wirkungsort transportiert werden und belasten dadurch nicht den gesamten Organismus. Dadurch kann auch vermieden werden, dass wie schlimmstenfalls zu befürchten ist, unerwünschte Nebenwirkungen der Wirkstoffe an allen Zellen auftreten.

Von Vorteil ist dabei die Ausgestaltung nach Anspruch 7, wonach durch diese Sequenzen eine gezielte Zufuhr von Wirkstoffen zu den Zellen des Auges, insbesondere zu Retinalzellen, ermöglicht wird. Weiters erweist sich als vorteilhaft, dass durch die Kombination mehrerer Oligopeptidsequenzen die Zielgenauigkeit des Transportsystems verbessert werden kann.

Die Weiterbildung des Transportsystems nach Anspruch 8 erweist sich als vorteilhaft, weil dadurch der Transport der Wirkstoffe direkt zu den Zielzellen, insbesondere zu Hautzellen, vorzugsweise zu Fibroblasten, erfolgen kann und es dadurch zu keiner Belastung anderer Zellen und somit anderer Organe kommt. Hier erweist sich als besonders vorteilhaft, dass durch eine Kombination mehrerer verschiedener Sequenzen der Transport der Wirkstoffe noch genauer erfolgen kann.

Dabei erweist sich nach Anspruch 9 als vorteilhaft, dass die Hybridpartikel nicht entlang eines Trägers angeordnet werden müssen sondern sich selbsttätig zu 3-dimensionalen Strukturen formieren. Vorteilhaft erweist sich weiters, dass durch die Anordnung der Hybridpartikel Wirkstoffe verpackt werden können und somit vor metabolischen Prozessen auf dem Weg zum Wirkungsort geschützt sind. Ein weiterer Vorteil ist, dass durch die Verpackung der Wirkstoffe in ein partikuläres Transportsystem immunologische Attacken und Abbauprozesse vermieden werden können und somit die Wirkstoffe in ihrer ursprünglichen Form am Wirkungsort ankommen.

Gemäß Anspruch 10 kann die Freisetzung der Mikronutrienten durch die Vernetzung der Hybridpartikel durch polymerisierbare Gruppen kontrolliert werden. Neben der Fähigkeit über einen langen Zeitraum in einem bestimmten Gewebe zu verweilen, stellt die exakte Kontrolle der physiko-chemischen Oberflächeneigenschaften, insbesondere der Konzentration und Präsentation eines Peptids an der Grenzfläche, ein weiteres wichtiges Kriterium für den erfolgreichen Einsatz von aktiven partikulären Transportsystemen für Wirkstoffe dar.

Vorteilhaft ist eine Weiterbildung des Transportsystem nach Anspruch 10, wonach der Transport vieler verschiedener Stoffe ermöglicht wird. Die Zufuhr eines dieser Stoffe bzw. einer Kombination dieser Stoffe liefert eine wichtige Basis für die Prophylaxe von Krankheiten und die Regeneration. Durch die Zufuhr dieser Stoffe kann der Verlauf von Krankheiten, vor allem von chronischen Krankheiten, günstig beeinflusst werden. Weiters ist von Vorteil, dass durch die Zufuhr mehrerer Mikronutrienten häufig eine wesentlich größere therapeutische Breite erzielt werden kann, weil oft aufgrund von Kombinationen mehrerer Mikronutrienten synergistische Effekte resultieren, wodurch sich die Pharmakologie der Mikronährstoffe zum Teil deutlich von jener anderer Arzneimittel unterscheidet. Durch die Zufuhr nur eines Mikronutrienten kann beispielsweise nachgewiesen werden, dass kein oxidativer Schutz erzielt wird, wohingegen eine Kombination aus zwei oder mehreren Mikronutrienten mit antioxidativen Eigenschaften antioxidative Wirkung zeigen.

Von Vorteil ist die Weiterbildung nach Anspruch 11, weil durch die Bereitstellung von Mikronutrienten die Prävention von Krankheiten und die Regeneration des Organismus unterstützt werden kann. Durch die orthomolekularen Eigenschaften der Mikronutrienten werden biochemische Reize gesetzt, die vom Organismus sinnvoll verwertet und beantwortet werden können, da es der Körper mit "Originalteilen" zu tun hat, d.h. mit Wirkstoffen, die ihm vertraut sind. Dadurch sind frühzeitige Interventionen im Energiestoffwechsel, eine Optimierung der Repairmechanismen, die Beseitigung von freien Radikalen und vieles andere möglich.

Gemäß Anspruch 12 und 13 ermöglicht das Transportsystem den zielgerichteten Transport und die Freisetzung für die bereitgestellten Mengen von Vitaminen, die der Organismus für lebenswichtige Funktionen benötigt, aber im Stoffwechsel nicht oder nicht in ausreichendem Umfang synthetisiert werden können und daher regelmäßig mit der Nahrung zugeführt werden müssen. Neben den spezifischen Funktionen sind beispielsweise Vitamine auch Bestandteile von Coenzymen, die den Zellstoffwechsel katalysieren.

Von Vorteil nach Anspruch 14 erweist sich, dass Mineralstoffe und Spurenelemente, die für Warmblüter essentiell sind, durch ein partikuläres Transportsystem zugeführt werden können. Die Elemente Natrium, Kalium, Magnesium und Kalzium sind in physiologischen Konzentrationen für die Aufrechterhaltung der Homöostase verantwortlich. Die Einführung synthetischer Diäten bei der Behandlung von Stoffwechselanomalien, die auf angeborenen genetischen Defekten beruhen, die Entwicklung der intravenösen Ernährung und die Dialysebehandlung von Patienten mit terminaler Niereninsuffizienz decken die iatrogenen Risiken auf, die die Bedeutung der alimentären Zufuhr, und falls diese nicht möglich ist, die Bedeutung der Supplementation dieser Elemente unterstreichen. Spurenelemente, die im Körper in minimalen Konzentrationen (weniger als 0,005 % des Körpergewichtes) vorkommen, kommt eine wichtige Rolle in der Physiologie des Menschen zu. Allerdings kann es mit der zunehmenden Entwicklung von synthetischen Nahrungsmitteln zu exzessiver Aufnahme dieser Elemente kommen. Es kommt daher bei alimentärer Zufuhr zu systemischen Belastungen des gesamten Organismus die Toxizitätserscheinungen hervorrufen, welche sich oft auch erst nach Jahren manifestieren können, und daher wird um so deutlicher die Notwendigkeit der zielgerichteten Zufuhr dieser Elemente dargestellt.

Weiters erweist sich nach Anspruch 15 von Vorteil, dass durch den gezielten Transport dieser Stoffe das allgemeine Wohlbefinden der Menschen verbessert werden kann. Beispielsweise ist Taurin an einer Reihe physiologischer Prozesse beteiligt, z.B. der Konjugation von Gallensäure, der Osmoregulation, der Detoxifikation von Xenobiotika, der Stabilisierung von Zellmembranen, der Steuerung des zellulären Kalziumstroms und der Modulation der neuronalen Erregbarkeit. Erniedrigte Taurinspiegel werden mit Netzhautdegenerationen, retartiertem Wachstum und Kardiomyopathie in Verbindung gebracht.

Gemäß der Weiterbildung des Verfahrens nach Anspruch 16 kann ein Mangel an essentiellen Aminosäuren, welcher zum Zusammenbruch der physiologischen Funktion des menschlichen Körpers führen kann, vermieden werden. Beispielsweise erhöht Arginin die Lymphozytenzahl und fördert allgemein die Bildung von immunkompetenten Zellen. Außerdem erhöht es die cytolytische Kapazität von Makrophagen und NK-Zellen. Es spielt auch eine wichtige Rolle bei der Wundheilung. Histidin wirkt als Antiallergikum und dient als Vorstufe von Histamin. Isoleuzin, Leuzin und Valin sind wichtige Bestandteile der Muskelproteine. Lysin ist der Hauptbestandteil von Collagen, Carnitinantikörpern, Hormonen und Enzymen, unterstützt die Wundheilung und fördert die Herpes simplex Heilung. Methionin ist ein Antioxidans, entgiftet die Leber und ist essentiell für die Selenwirkung (Absorption, Transport, Bioverfügbarkeit). Phenylalanin besitzt antidepressive Wirkung und verlängert die Wirkung und erhöht die Aktivität von Endorphinen. Threonin ist ein lipotroper Faktor. Tryptophan ist wichtig für die Synthese von Vitamin B3 und ist ein Vorstufe des Serotonins und Melatonins (Schlafrhythmus).

Gemäß der Ausgestaltung nach Anspruch 17, ist eine optimale Versorgung von essentiellen Fettsäuren vor allem während des Wachstums und in der zweiten Lebenshälfte von außerordentlicher Bedeutung für den Organismus. So erhalten sie z.B. die Elastizität der Membranen aller Körperzellen sowie der Mitochondrien und sorgen für eine Zellverjüngung. Sie kommen in den Gonaden vor und bilden die Bausteine für die körpereigene Hormonproduktion im endokrinen Drüsensystem aber auch im Zellgewebe. Eine herausragende Rolle unter den essentiellen Fettsäuren spielen die Linol- und die α-Linolensäure. Sie dienen als Strukturelement der Zellmembran und steuern mit den aus ihnen entstandenen Produkten, wie z.B. Prostaglandine, Thromboxan und Leukotriene, viele lebenswichtige Prozesse im Organismus. Die Arachidonsäure kommt nur in tierischen Fetten vor und ist ein Ausgangsprodukt für die Prostaglandinsynthese. Durch die gezielte Zufuhr von Arachidonsäure zu Prostaglandinbildungsstätten über den Cyclooxygenase-1 Metabolisierungsweg wird eine systemische Überversorgung an Arachidonsäure mit einem negativen Einfluss auf rheumatische Gelenksentzündungen vermieden.

Die Aufgabe der Erfindung wird eigenständig durch eine Verfahren entsprechend den Merkmalen im Kennzeichenteil des Anspruches 18 gelöst. Vorteilhaft daran ist, dass eine systemische Belastung des Organismus vermieden werden kann, weil der Wirkstoff nur in der Zielzelle freigesetzt wird. Dadurch können systemische Nebenwirkungen durch zu hohe Konzentrationen der Wirkstoffe in anderen Zellen als den Zielzellen vermieden werden.

Die Aufgabe der Erfindung wird eigenständig auch durch die Verwendung des Transportsystems entsprechend dem Anspruch 19 gelöst. Von Vorteil dabei erweist sich, dass ein präventiver und regenerativer Einfluss auf den Organismus bewirkt werden kann.

Vorteilhaft nach Anspruch 20 erweist sich, dass Ernährungsdefizite, die lokale Beeinträchtigungen in der Aufrechterhaltung des physiologischen Gleichgewichts im Organismus verursachen, reduziert bzw. behoben werden können.

Nach Anspruch 21 erweist sich als vorteilhaft, dass das Transportsystem durch die topische Applikation direkt an den Wirkungsort gebracht werden kann und der Transport des Wirkstoffes durch die Zellmembran und nicht nur über das Blut- bzw. Lymphsystem erfolgt.

Als vorteilhaft nach Anspruch 22 erweist sich, dass das Transportsystem sowohl in der pharmazeutischen, kosmetischen als auch in der Nahrungsmittelindustrie Anwendung findet.

Die Erfindung wird im nachfolgenden anhand der in den Zeichnungen dargestellten Ausführungsbeispielen näher erläutert.

Es zeigen jeweils in vereinfachter Darstellung:
- Fig. 1: einen Hybridpartikel;
- Fig. 2: einen Hybridpartikel mit polymerisierbarer Gruppe;
- Fig. 3: ein Transportsystem.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

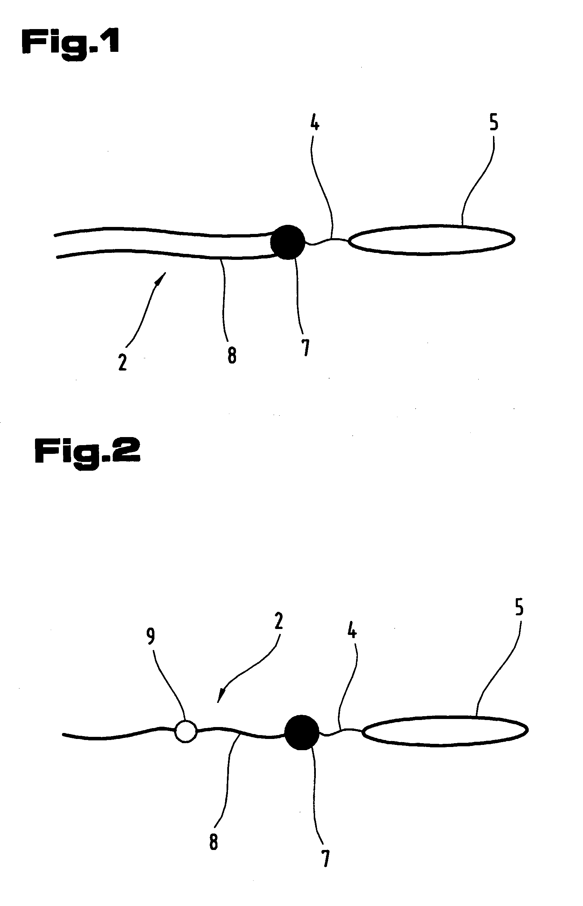

In den gemeinsam beschrieben Fig. 1 bis 3 wird ein Transportsystem 1 in biologischen System dargestellt.
Fig. 1 zeigt die Struktur eines bioaktiven Bausteins von Transportsystemen 1. Das Transportsystem 1 wird von Hybridpartikeln 2, bestehend aus einem Lipidmolekül 3, einer Spacereinheit 4 und einem Peptid 5 gebildet. Die Sequenz des bioaktiven Oligopeptids 6 wird über eine kurze Spacereinheit 4 an die polare Kopfgruppe 7 des synthetischen oder natürlichen Lipidmoleküls 3 gekoppelt.
Fig. 2 zeigt die schematische Darstellung eines Hybridpartikels 2, welches in die unpolare Kohlenwasserstoffkette 8 des Lipidmoleküls 3 eine polymerisierbare Gruppe 9 eingebracht hat.

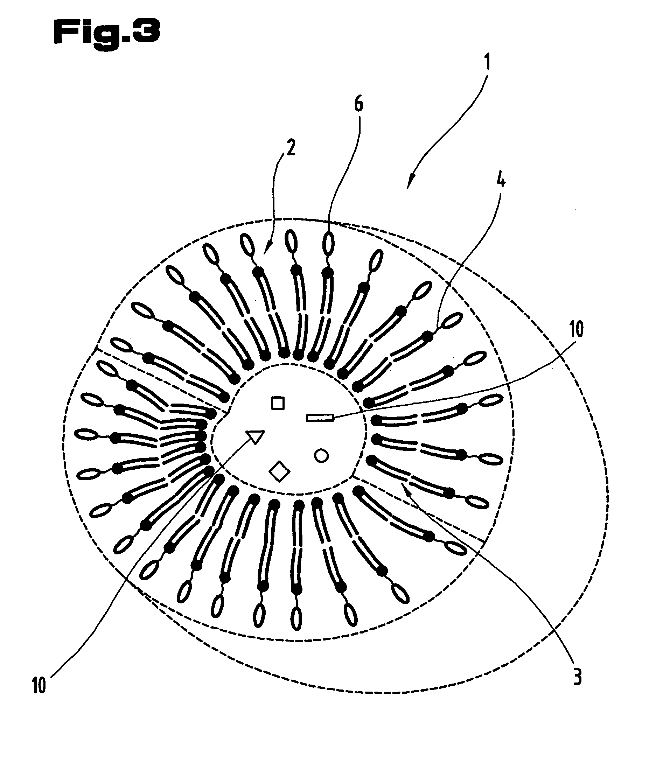

Fig. 3 zeigt einen Schnitt durch das Transportsystem 1, wobei der Umriss des Transportsystems 1 durch eine strichlierte Linie dargestellt ist. Das Transportsystem 1 besteht aus einer Doppelschicht von Hybridpartikeln 2 und Lipidmolekülen 3, wobei die Hybridpartikel 2 die äußere Schicht der Doppelschicht und die Lipidmoleküle 3 die innere Schicht bilden. Die Hybridpartikel 2 und Lipidmoleküle 3 formieren sich zu einer räumlichen Struktur und in ihrem Inneren sind Wirkstoffe 10, insbesondere Mikronutrienten, vorzugsweise hydrophobe Mikronutrienten, eingeschlossen. In einer weiteren Ausführungsform kann die äußere Schicht des Transportsystems 1 auch teilweise von Lipidmolekülen 3 gebildet werden, wobei die Hybridpartikel 2 und die Lipidmoleküle 3 in einer beliebigen Reihenfolge angeordnet sein können, wie z.B. dass nur an jeder zweiten oder dritten polaren Kopfgruppe 7 eines Lipidmoleküls 3 ein Oligopeptid 6 angeordnet ist. In beiden Ausführungsformen kann in die Kohlenwasserstoffkette 8 des Lipidmoleküls 3 selbst oder des Hybridpartikels 2 auch eine polymerisierbare Gruppe 9 angebracht sein.

Als Wirkstoffe 10 werden insbesondere Mikronutrienten, wie Provitamine, Vitamine, Mineralstoffe und Spurenelemente, Aminosäuren, Fettsäuren, Polyphenole, Hormone und Organextrake bzw. deren Syntheseprodukte, wie z.B. Pankreatin, Gallensäure, Knorpelgrundsubstanz, etc., aber selbstverständlich auch Farbstoffe, wie z.B. Kontrastmittel, die für bildgebende Verfahren in medizinischen Untersuchungen zielgerichtet transportiert werden müssen, verstanden.

Hybridpartikel 2 aus Lipidmolekülen 3, welche mit bestimmten Peptiden 5 an der polaren Kopfgruppe 7 modifiziert sind, können durch die symmetrische Anordnung der unpolaren Kohlenwasserstoffketten 8 zum Aufbau von selbstorganisierten dreidimensionalen Strukturen, insbesondere Transportsysteme 1, wie z.B. Liposomen, Mizellen und Öl-in-Wasser-Emulsionen, verwendet werden. Es ist eine exakte Kontrolle wichtiger physikalisch-chemischer Oberflächeneigenschaften, wie z.B. die Oberflächenkonzentration des Peptids 5, möglich.

Die Oligopeptidsequenz ist eine Steuerungssequenz zur zielgerichteten Adressierung des Hybridpartikels 2 und/oder des Transportsystems 1. Solch bioaktive Peptidsequenzen, welche kontrolliert auf der Oberfläche dieser Systeme gebunden sind, stellen die Grundlage für eine effiziente sowie spezifische Zuführung der Partikel zu bestimmten Zellen in bestimmten Geweben sowie eine Verhinderung von nicht spezifischen Wechselwirkungen mit Proteinen dar. Als bioaktive Oligopeptide 6 werden Liganden adaptiert, welche typischerweise in Signalproteinen des extrazellulären Raumes gefunden werden.

Insbesondere ermöglicht die Verwendung von Transportsystemen 1 aus Hybridpartikeln 2 mit Oligopeptiden 6 mit zumindest einer der Sequenzen Gly-Arg-Gly-Asp-Ser-Pro (SEQ ID NO: 1), welche eine Fibronektinrezeptorbindungsstelle bildet, Tyr-Ile-Glu-Ser-Arg (SEQ ID NO: 2), welche eine Lamininrezeptorbindungsstelle ist, und/oder Ala-Asp-Gly-Glu-Ala (SEQ ID NO: 3), welche eine Kollagenrezeptorbindungsstelle darstellt, einen zielgerichteten Transport von Wirkstoffen 10 zu Zellen der Haut, insbesondere zu Fibroblasten.

Für den zielgerichteten Transport von Mikronutrienten zu Zellen des Auges, insbesondere zu retinalen Zellen, eignen sich Hybridpartikel 2 mit Oligopeptiden 6 mit zumindest einer der Sequenzen Val-Arg-Leu-Leu-Asn-Asn (SEQ ID NO: 4), Val-Arg-Leu Leu-Asn-Asn-Trp-Asp (SEQ ID NO: 5), Gly-Arg-Val-Arg-Leu-Leu-Asn-Asn (SEQ ID NO: 6), welche Bindungsstellen für das Retinolbindungsprotein an RP 65 charakterisieren. Des weiteren eigenen sich Hybridpartikel 2 mit Oligopeptiden 6 mit zumindest einer der Sequenzen Met-Thr-Ala-Gly-Ala-Gly (SEQ ID NO: 7), Leu-Ser-Gly-Ala-Leu-Arg (SEQ ID NO: 8), Ile-Val-Ala-Ile-Leu-Ile-Cys-Ile-Leu-Ile-Leu-Leu-Thr-Met-Val-Leu-Leu-Phe-Val-Met-Trp-Met (SEQ ID NO: 9), wobei ein beliebiger Abschnitt aus der Aminosäurenabfolge der SEQ ID NO: 9 als Bindungsstelle ausgewählt werden kann, wie z.B. Ile-Val-Ala-Ile-Leu-Ile-Cys-Ile-Leu-Ile-Leu-Leu (SEQ ID NO: 10), Ile-Val-Ala-Ile-Leu-Ile-Cys-Ile-Leu-Ile-Leu-Leu-Thr-Met-Val-Leu-Leu-Phe (SEQ ID NO: 11), Ile-Val-Ala-Ite-Leu-Ile (SEQ ID NO: 12), Cys-Ile-Leu-Ile-Leu-Leu (SEQ ID NO: 13), Thr-Met-Val-Leu-Leu-Phe (SEQ ID NO: 14) und/oder Leu-Phe-Val-Met-Trp-Met (SEQ ID NO: 15), welche Bindungsstellen für R-Cadherin für die Zell-Zell-Adhäsion im speziellen für die Retina sind. In weiteren Ausführungsformen sind sowohl für den zielgerichteten Transport zu Zellen der Haut als auch des Auges mehrere Kombinationen verschiedener Sequenzen der jeweils zugeordneten Oligopeptide 6 möglich.

Für den Aufbau funktionaler Transportsysteme 1, welche aus selbstorganisierenden Hybridpartikeln 2 bzw. Lipidmolekülen 3 zusammengesetzt sind, wird folgende Strategie verfolgt, dass das Peptid 5 zuerst an eine Spacereinheit 4 und anschließend das Oligopeptid 6 gebunden an die Spacereinheit 4 gemeinsam an die polare Kopfgruppe 7 von Lipidmolekülen 3 gekoppelt wird. In einem zweiten Schritt werden die fertigen ligandenmodifizierten Hybridpartikel 2 zu Transportsystemen 1 zusammengesetzt. Diese Strategie ermöglicht eine exakte Kontrolle der Oberflächenkonzentration des Oligopeptids 6. Per Definition sitzen die Oligopeptide 6 hier alle Außen und durch die Wahl der Konzentrationen an Lipidmolekülen 3 und ligandenmodifizierten Lipidmolekülen 3 können verschiedene Emulsionsphasen kontrollierbar, hinsichtlich der hydrodynamischen Abmessungen und der physikalisch-chemischen Oberflächeneigenschaften eingestellt werden.

Mit Hilfe dieses Baukonzeptes ist es möglich, dreidimensionale bioaktive Transportsysteme 1 zu bauen, welche aktiv mit bestimmten Zielzellen und somit Gewebetypen kommunizieren.

Zur beabstandeten Anordnung des Oligopeptids 6 am Lipidmolekül 3 können als Spacereinheit 4 verschiedene Substanzen, wie z.B. eine Abfolge von Aminosäuren oder chemisch inerte Substanzen, wie z. B. Nanopartikel, wie Karbonanotubes, Nanofäden, Kolloide, etc., eingesetzt werden.

Neben der spezifischen Zuführung wird das kontrollierte Freisetzen von Mikronutrienten aus den Partikeln durch den Einbau von polymerisierbaren Gruppen in die Molekülstruktur der verwendeten Hybridpartikel 2 und somit durch die Vernetzung der Lipidschichten in zwei Dimensionen erreicht. Parameter, wie z.B. die Größe und Form (hydrodynamische Abmessungen) der Hybridpartikel 2, physikalisch chemische Oberflächeneigenschaften sowie die Stabilität des Transportsystems 1 sind für die gezielte Zufuhr von Mikronutrienten jeweils von großer Bedeutung. Die Polymerisation innerhalb der selbstorganisierten Schichten führt zu einer Stabilisierung und kann zur Kontrolle der Freisetzung von eingeschleusten Mikronährstoffen verwendet werden. Eine Kontrolle der Mikronährstoffabgabe zielt darauf hin ab, dass bestimmte Mikronährstoffe in dem Zielgewebe nicht nur angereichert werden, sondern auch über einen längeren Zeitraum in einer hohen Konzentration konstant bleiben.

Der Aufbau einer solchen Barriere erfolgt dadurch, dass man die Lipidmoleküle 3, welche innerhalb der Mono- bzw. Doppellagen lateral frei diffundieren und so einen regen Austausch von Molekülen innerhalb und außerhalb der Partikel ermöglicht, fixiert. Dies kann z.B. durch den Einbau von polymerisierbaren Gruppen in die Hybridpartikel 2 und anschließende Polymerisation bzw. Teilpolymerisation in zwei Dimensionen (innerhalb der Lipidschichten) erfolgen. Beispielsweise können Diacetylen-Lipide verwendet werden, welche durch Polymerisation die Bildung von selbstorganisierten Strukturen, wie z.B. langzeitstabile Vesikel, stabile Röhren mit sehr hohen Persistenzen, helikale Mikrostrukturen, etc., ermöglichen Diacetylen-Lipide polymerisieren unter dem Einfluss von UV-Licht (λ = 254 nm) in einer Additionsreaktion. Diese Polymerisationsreaktion ist photochemisch kontrollierbar und die Diacetylen-Lipide polymerisieren fast ausschließlich innerhalb einer kristallinen Lipidphase. Hierbei entsteht ein Polymer, welches über ein ausgedehntes, konjugiertes Elektronensystem verfügt und in Folge dessen, Licht in visuellem Bereich absorbiert. Die Lage der Absorption ist abhängig von der Struktur (Konformation) des Polymers und kann durch verschiedene Parameter, wie z.B. eine Temperaturerhöhung und/oder mechanischen Stress, wie z.B. die Anbindung eines Liganden an einen entsprechenden Rezeptor, beeinflusst und verschoben werden.

Die Polymerisation ergibt langzeitstabile vesikuläre Transportsysteme 1, die ein Absorptionsmaximum bei λ = 640 nm haben. Während Transportsysteme 1, welche keine Liganden auf der Oberfläche besitzen, keine Änderung der Absorption zeigen, so ist erkennbar, dass peptidmodifizierte Transportsysteme 1 eine deutliche Wechselwirkung mit den Zellen eingehen und das Absorptionsmaximum sich signifikant verschiebt. Es können auch hydrophobe Teststoffe in die Membran des Transportsystems 1 vor der Polymerisation eingeschleust werden, und die Freisetzung dieser Teststoffe in Abhängigkeit der Menge an polymerisierbaren Lipidmolekülen 3 in der Membran gesteuert werden.

Es können auch teilpolymerisierte Transportsysteme 1 durch die Zugabe von nicht polymerisierbaren Hybridpartikeln 2 hergestellt werden, die in verschiedenen physiologischen Umgebungen (pH-Wert, Ionenstärke, etc.) stabil sind. Zur sterischen Abschirmung (Unterbindung von nicht spezifischen Wechselwirkungen, z.B. mit Proteinen) werden Lipopolymere eingebaut. Zur besseren Analyse dieser Strukturen können auch floureszierende Lipide eingebaut werden. Insbesondere die Länge und die Konzentration der eingesetzten Lipopolymere beeinflusst die Bioaktivität des Transportsystems 1. Durch optische (Lichtstreuung, UV-Absorbtion, Floureszenz) und mikroskopische Methoden (ASM, Transmissionselektronenmikroskopie (TEM), Floureszenz) können die morphologischen und physiko-chemischen Eigenschaften und deren Auswirkungen auf die Parameter, wie z.B. die Effizienz der Beladung mit Mikronutrienten, die Stabilität in verschiedensten Umgebungen und die kontrollierte Freisetzung und der zielgerichtete Transport charakterisiert werden.

Polymerisierbare Hybridpartikel 2 können auch zur Herstellung von thermodynamisch stabilen Mikroemulsionen eingesetzt werden um so möglichst kleine bioaktive Transportsysteme 1 mit einem Durchmesser mit einer unteren Grenze von 5 nm, insbesondere 10 nm, vorzugsweise 15 nm und einer oberen Grenze von 180 nm, vorzugsweise 160 nm, insbesondere 140 nm, herzustellen. Vorteilhaft hat sich auch ein Bereich mit einer unteren Grenze von 20 nm, insbesondere 30 nm und einer oberen Grenze von 120 nm, insbesondere 100 nm erwiesen. Diese thermodynamisch stabilen Mikroemulsionen verfügen über ein größt mögliches, hydrophobes Transportvolumen. Diese Emulsionen können neben den Hybridpartikeln 2 auch noch polymerisierbare Lipide und Lipopolymere sowie weitere hydrophobe Komponenten enthalten. Das Phaseverhalten dieser Öl-in-Wasser-Emulsion ist eine Funktion der Konzentration der einzelnen Parameter der Temperatur und der Ionenstärke des Mediums mit dem Ziel, eine thermodynamisch stabile Emulsionsphase herzustellen.

Auch eine Übertragung des Bauprinzips auf "natürliche" Systeme, so z.B. der Aufbau von peptidmodifizierten Phospho-, Glyco-, Sphingolipiden, Steroiden und/oder Polyisoprenoiden ist möglich. Hybridpartikel 2 können demnach auch auf der Basis von natürlichen Phospholipiden synthetisiert und charakterisiert werden. Hier wird beispielsweise das Phospholipid direkt an das orthogonal geschützte Peptid 5 gebunden und in einem finalen Schritt das Molekül sowie alle Schutzgruppen von der Festphase abgespalten. Die Aufreinigung erfolgt mittels Hochleistungschromatographie (HPLC). Nach der Charakterisierung mittels Fourier-Transformation Infrarot Spektroskopie (FTIR), Kernmagnetresonanzspektroskopie (NMR) oder Massenspektroskopie (MS) werden die hergestellten modifizierten Phospholipide zusammen mit nicht modifizierten Lipiden zum Aufbau von bioaktiven Transportsystemen 1 und Emulsionen eingesetzt. Als Liganden können die selben Oligopeptide 6, wie oben charakterisiert, verwendet werden.

Bioaktive planare Oberflächen, welche über die Selbstorganisation dieser synthetischen Hybridpartikel 2 und anschließender Modifikation von Festkörpersubstraten hergestellt werden, zeigen ein hohes Potential zur Adhäsion. Neben dreidimensionalen Strukturen werden auch planare Oberflächen mit polymerisierten Monolagen der Hybridpartikel 2 modifiziert. Es können Zellen auf diesen Oberflächen adhärent werden und spreiten. Ferner können diese Oberflächen beliebig oft mit Zellen wieder besiedelt werden, da sie durch die Polymerisation nicht mehr von der Oberfläche ablösbar sind und somit Zellen relativ einfach mehrmals entfernt werden können. Es ist auch eine Quantifizierung der Ergebnisse möglich.

Transportsysteme 1 für Wirkstoffe 10 können zur Behandlung von Erkrankungen in Folge von unzureichender Versorgung mit Mikronutrienten eingesetzt werden. Das Transportsystem 1 kann auch Wirkstoffe 10 für kosmetische Anwendungen transportieren und kann sowohl oral als auch topisch appliziert werden.

### Vergleichsbeispiel 1

### fettlösliche Vitamine (Tocopherole):

Es können Hybridpartikel 2 mit Oligopeptidsequenzen wie z.B. Gly-Arg-Gly-Asp-Ser-Pro (SEQ ID NO: 1) als Transportsystem 1 für Tocopherole für Hautzellen, insbesondere Fibroblasten, verwendet werden. Durch die gezielte Zufuhr von Mikronutrienten werden sowohl präventive als auch regenerative Prozesse, wie z.B. die Gewebserhaltung und Wundheilung der Haut unterstützt. Bei Verbrennungen und bei der Wundheilung kommt es zu einer Konzentrationsabnahme von Antioxidantien. Dies gilt insbesondere für Tocopherole, die mengenmäßig die wichtigsten fettlöslichen Vitamine der Haut darstellen. Gerade bei Verbrennungen ist jedoch die systemische Zufuhr von Mikronutrienten besonders schlecht bzw. gar nicht mehr vorhanden. Durch die zielgerichtete Zufuhr von Tocopherolen während der regenerativen Behandlung können diese Nährstoffe direkt in den Zielzellen angereichert und kontrolliert freigesetzt werden. Die Wirkstoffe 10 können aufgrund der spezifischen Liganden in verschiedenen Hautschichten festgehalten, akkumuliert und freigesetzt werden und dadurch lokal wirken. Zum anderen wird auch das Penetrationsverhalten aufgrund der geringen Partikelgröße positiv beeinflusst. Als Modell für die Untersuchung können 3-dimensionale Hautrnodelle verwendet werden.

### Vergleichsbeispiel 2

### Carotinoide

Für die Verpackung und den zielgerichteten Transport von Carotinoiden zu Zellen der Retina wird ein Transportsystem 1 umfassend Hybridpartikel 2 mit der Oligopeptidsequenz Val-Arg-Leu-Leu-Asn-Asn (SEQ ID NO: 4) verwendet. Durch die gezielte Zufuhr von Mikronutrienten werden präventive Prozesse , wie z.B. die Vermeidung von alterabhängiger Makuladegeneration (AMD), unterstützt. Bei AMD konnte nachgewiesen werden, dass in der Retina zu geringe Konzentrationen der Carotinoide Lutein und Zeaxanthin vorkommen. Durch die Verpackung von Carotinoiden in partikuläre Systeme werden die Provitamine zielgerichtet zu den Zielzellen transportiert. Zelllinien des retinalen Epithels können als Modellsystem für das Auge verwendet werden.

Der Ordnung halber sei abschließend darauf hingewiesen, dass zum besseren Verständnis des Aufbaus des Transportsystems 1 für Wirkstoffe 10 dieses bzw. deren Bestandteile teilweise unmaßstäblich und vergrößert dargestellt wurden.

Die den eigenständigen erfinderischen Lösungen zugrundeliegende Aufgabe kann der Beschreibung entnommen werden.

Vor allem können die einzelnen in den Fig. 1, 2, 3 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Transportsystem
- 2: Hybridpartikel
- 3: Lipidmolekül
- 4: Spacereinheit
- 5: Peptid

- 6: Oligopeptid
- 7: polare Kopfgruppe
- 8: unpolare Kohlenwasserstoffketten
- 9: polymerisierbare Gruppe
- 10: Wirkstoffe

## Patentansprüche

1. Transportsystem für Wirkstoffe umfassend Hybridpartikel aus zumindest einer Schicht von Lipidmolekülen und zumindest einen über eine Spacereinheit verbundenen Liganden, der ein Peptid ist, **dadurch gekennzeichnet, dass** zumindest eine polymerisierbare Gruppe in die Hybridpartikel eingebaut ist.

2. Transportsystem nach Anspruch 1, **dadurch gekennzeichnet, dass** die Spacereinheit von Aminosäuren, einer chemisch inerten Substanz, wie Nanopartikel, ausgewählt aus einer Gruppe umfassend Karbonnanotubes, Nanofäden oder Kolloide, gebildet ist.

3. Transportsystem nach Anspruch 1 oder 2, **dadurch gekennzeichnet, dass** die Lipidmoleküle polymerisierbare Lipide und/oder "natürliche" Lipide, wie z.B. Steroide, Glycolipide, Phospholipide, Sphingolipide, Polyisoprenoide, sind.

4. Transportsystem nach einem der vorhergehenden Ansprüche, **dadurch gekennzeichnet, dass** das Peptid ein Oligopeptid ist.

5. Transportsystem nach Anspruch 4, **dadurch gekennzeichnet, dass** das Oligopeptid eine Länge, ausgewählt aus einem Bereich mit einer unteren Grenze von 4 Aminosäuren,
vorzugsweise 5 Aminosäuren, insbesondere 6 Aminosäuren und einer oberen Grenze von 18 Aminosäuren, vorzugsweise 20 Aminosäuren, insbesondere 22 Aminosäuren aufweist.

6. Transportsystem nach Anspruch 4 oder 5, **dadurch gekennzeichnet, dass** die Oligopeptidsequenz komplementär zur Sequenz eines Rezeptors an einer Zelle ist.

7. Transportsystem nach einem der Ansprüche 4 bis 6, **dadurch gekennzeichnet, dass** das Oligopeptid eine Sequenz, ausgewählt aus einer Gruppe umfassend die Sequenzen Gly-Arg-Gly-Asp-Ser-Pro (SEQ ID NO: 1), Tyr-Ile-Glu-Ser-Arg (SEQ ID NO: 2) und/oder Ala-Asp-Gly-Glu-Ala (SEQ ID NO: 3), aufweist.

8. Transportsystem nach einem der Ansprüche 4 bis 6, **dadurch gekennzeichnet, dass** das Oligopeptid eine Sequenz, ausgewählt aus einer Gruppe umfassend die Sequenzen
Val-Arg-Leu-Leu-Asn-Asn (SEQ ID NO: 4), Val-Arg-Leu Leu-Asn-Asn-Trp-Asp (SEQ ID NO: 5), Gly-Arg-Val-Arg-Leu-Leu-Asn-Asn (SEQ ID NO: 6), Met-Thr-Ala-Gly-Ala-Gly (SEQ ID NO: 7), Leu-Ser-Gly-Ala-Leu-Arg (SEQ ID NO: 8), Ile-Val-Ala-Ile-Leu-Ile-Cys-Ile-Leu-Ile-Leu-Leu-Thr-Met-Val-Leu-Leu-Phe-Val-Met-Trp-Met (SEQ ID NO: 9), Ile-Val-Ala-Ile-Leu-Ile-Cys-Ile-Leu-Ile-Leu-Leu (SEQ ID NO: 10), Ile-Val-Ala-Ile-Leu-Ile-Cys-Ile-Leu-Ile-Leu-Leu-Thr-Met-Val-Leu-Leu-Phe (SEQ ID NO: 11), Ile-Val-Ala-Ile-Leu-Ile (SEQ ID NO: 12), Cys-Ile-Leu-Ile-Leu-Leu (SEQ ID NO: 13), Thr-Met-Val-Leu-Leu-Phe (SEQ ID NO: 14) und/oder Leu-Phe-Val-Met-Trp-Met (SEQ ID NO: 15), aufweist.

9. Transportsystem nach einem der vorhergehenden Ansprüche, **dadurch gekennzeichnet, dass** die Hybridpartikel 3-dimensionale Strukturen, wie z.B. Vesikel, Mikrosphären, Nanopartikel, Tubes, bilden.

10. Transportsystem nach einem der vorhergehenden Ansprüche, **dadurch gekennzeichnet, dass** der Wirkstoff zumindest ein Mikronutrient ist.

11. Transportsystem nach Anspruch 10, **dadurch gekennzeichnet, dass** der zumindest eine Mikronutrient zumindest ein Stoff, ausgewählt aus einer Gruppe umfassend Provitamine, Vitamine, Mineralstoffe und Spurenelemente, Aminosäuren, Fettsäuren, Polyphenole, Hormone und Organextrake bzw. deren Syntheseprodukte, wie z.B. Pankreatin, Gallensäure, Knorpelgrundsubstanz, ist.

12. Transportsystem nach Anspruch 10 oder 11, **dadurch gekennzeichnet, dass** das Vitamin ausgewählt aus einer Gruppe umfassend natürliche und synthetische Verbindungen mit Retinoidstruktur (Vitamine A), Vitamin-B-Komplex, Ascorbinsäuren (Vitamine C), Calciferole (Vitamine D), Tocopherole (Vitamine E), Vitamine K, Flavanoide und Biotin, ist.

13. Transportsystem nach einem der Ansprüche 10 bis 12, **dadurch gekennzeichnet, dass** das zumindest eine Vitamin, ausgewählt aus einer Gruppe umfassend Retinol, Retinyl-Acetat, Retinyl-Palmitat, 3,4-Didehydroretinol (Vitamin A2), Retinal, Retinsäure und Provitamine, wie z.B. α-, β-, γ-Carotin, Lutein, Zeaxanthin, Thiamin (Vitamin B1) bzw. Thiaminhydrochlorid bzw. Thiaminmonomitrat, Riboflavin (Vitamin 82) bzw. Natrium-Riboflavin-5-Phosphat, Niacin (Vitamin B3) bzw. Nikotinsäure bzw. Neacin, Pantothensäure (Vitamin 85) bzw. Calcium-D-Pantothenat bzw. Natrium-D-Pantothenat bzw. D-Panthenol, Pyridoxin (Vitamin B6) bzw. Pyridoxinhydrochlorid bzw. Pyridoxin-5-Phosphat bzw. Pyridoxindipalmitat bzw. Pyridoxalphosphat, Folsäure (Vitamin B9) bzw. Pteroylglutaminsäure, Cobalamin (Vitamin B 12) bzw. Cyanocobalamin bzw. Hydroxycobalamin, Biotin, Cholin, Inosit und p-Aminobenzoesäure, L-Ascorbinsäure, Natrium-L-Ascorbat, Calcium-L-Ascorbat, Kalium-L-Ascorbat und L-Ascorbyl-6-Palmitat, Ergocalciferol (Vitamin D2), Cholecalciferol (Vitamin D3), 1,25-Dihydroxycholecalciferol und die Provitamine Ergosterol bzw. 7-Dehydrocholesterol, D-α-tocopherol, DL-α-Tocopherol, D-α-Tocopherylacetat, DL-α-Tocopherylacetat und D-α-Tocopherylsäuresuccinat, Phylloquinon (Vitamin K1), Menoquinon (Vitamin K2), Menadion (Vitamin K3) und Menadionhydroxiquinon (Vitamin K4), ist.

14. Transportsystem nach Anspruch 10 oder 11, **dadurch gekennzeichnet, dass** der zumindest eine Mineralstoff bzw. das zumindest eine Spurenelement in der Reihenfolge ihrer Bedeutung für den Organismus, ausgewählt aus einer Gruppe umfassend Na, K, Mg, Ca, Fe, I, Cu, Mn, Zn, Co, Mo, Se, Cr, F, Si, Ni, As, Sn, V, P, Cl, B, Al und Br, ist.

15. Transportsystem nach Anspruch 10 oder 11, **dadurch gekennzeichnet, dass** die zumindest eine Komponente, ausgewählt aus einer Gruppe umfassend Coenzym Q-10, Quercetin, Bromelain, Inositol, Cholin, Pycnogenol, Carnitin, Taurin, Mesoinosit, ist.

16. Transportsystem nach Anspruch 10 oder 11, **dadurch gekennzeichnet, dass** die zumindest eine essentielle Aminosäure, ausgewählt aus einer Gruppe umfassend Histidin, Isoleuzin, Leuzin, Lysin, Methionin, Phenylalanin, Threonin, Tryptophan, Valin und Arginin, ist.

17. Transportsystem nach Anspruch 10 oder 11, **dadurch gekennzeichnet, dass** die zumindest eine Fettsäure, ausgewählt aus einer Gruppe umfassend Linolsäure, Linolensäure und Arachidonsäure, ist.

18. Verfahren zum Transport von Wirkstoffen, **dadurch gekennzeichnet, dass** das Transportsystem nach einem der Ansprüche 1 bis 17 verwendet wird.

19. Transportsystem nach einem der Ansprüche 1 bis 17 zur Verwendung als Arzneimittel.

20. Verwendung des Transportsystems nach Anspruch 19 zur Herstellung eines Arzneimittels zur Behandlung von Ernährungsdefiziten.

21. Verwendung des Transportsystems nach Anspruch 20 zur topischen und oralen Applikation.

22. Verwendung des Transportsystems nach einem der Ansprüche 1 bis 17, **dadurch gekennzeichnet, dass** es in der pharmazeutischen, kosmetischen und Nahrungsmittelindustrie angewendet wird.

## Claims

1. Transport system for active substances containing hybrid particles comprising at least one layer of lipid molecules and at least one ligand, which is a peptide, bonded via a spacer unit, **characterised in that** at least one polymerisable group is incorporated in the hybrid particles.

2. Transport system as claimed in claim 1, **characterised in that** the spacer unit is provided in the form of amino acids, a chemically inert substance, such as nano-particles, selected from a group comprising carbon nano-tubes, nano-threads or colloids.

3. Transport system as claimed in claim 1 or 2, **characterised in that** the lipid molecules are polymerisable lipids and/or "natural" lipids, such as steroids, glycolipids, phospholipids, sphingolipids, polyisoprenoids, for example.

4. Transport system as claimed in one of the preceding claims, **characterised in that** the peptide is an oligopeptide.

5. Transport system as claimed in claim 4, **characterised in that** the oligopeptide has a length selected from a range with a lower limit of 4 amino acids, preferably 5 amino acids, in particular 6 amino acids, and an upper limit of 18 amino acids, preferably 20 amino acids, in particular 22 amino acids.

6. Transport system as claimed in claim 4 or 5, **characterised in that** the oligopeptide sequence is complementary to the sequence of a receptor on a cell.

7. Transport system as claimed in one of claims 4 to 6, **characterised in that** the oligopeptide has a sequence selected from a group comprising the sequences Gly-Arg-Gly-Asp-Ser-Pro (SEQ ID NO: 1), Tyr-Ile-Glu-Ser-Arg (SEQ ID NO: 2), and/or Ala-Asp-Gly-Glu-Ala (SEQ ID NO: 3).

8. Transport system as claimed in one of claims 4 to 6, **characterised in that** the oligopeptide has a sequence selected from a group comprising the sequences Val-Arg-Leu-Leu-Asn-Asn (SEQ ID NO: 4), Val-Arg-Leu-Leu-Asn-Asn-Trp-Asp (SEQ ID NO: 5), Gly-Arg-Val-Arg-Leu-Leu-Asn-Asn (SEQ ID NO: 6), Met-Thr-Ala-Gly-Ala-Gly (SEQ ID NO: 7), Leu-Ser-Gly-Ala-Leu-Arg (SEQ ID NO:
8), Ile-Val-Ala-Ile-Leu-Ile-Cys-Ile-Leu-Ile-Leu-Lei-Thr-Met-Val-Leu-Leu-Phe-Val-Met-Trp-Met (SEQ ID NO: 9), Ile-Val-Ala-Ile-Leu-Ile-Cys-Ile-Leu-Ile-Leu-Leu (SEQ ID NO: 10), Ile-Val-Ala-Ile-Leu-Ile-Cys-Ile-Leu-Ile-Leu-Leu-Thr-Met-Val-Leu-Leu-Phe (SEQ ID NO: 11), Ile-Val-Ala-Ile-Leu-Ile (SEQ ID NO: 12), Cys-Ile-Leu-Ile-Leu-Leu (SEQ ID NO: 13), Thr-Met-Val-Leu-Leu-Phe (SEQ ID NO: 14) and/or Leu-Phe-Val-Met-Trp-Met (SEQ ID NO: 15).

9. Transport system as claimed in one of the preceding claims, **characterised in that** the hybrid particles form 3-dimensional structures, such as vesicles, micro-spheres, nano-particles, tubes, for example.

10. Transport system as claimed in one of the preceding claims, **characterised in that** the active substance is at least one micro-nutrient.

11. Transport system as claimed in claim 10, **characterised in that** the at least one micro-nutrient is at least one substance selected from a group comprising provitamins, vitamins, minerals and trace elements, amino acids, fatty acids, polyphenols, hormones and organ extracts or their synthesis products, such as pancreatin, galenic acid, cartilagenous base substance for example.

12. Transport system as claimed in claim 10 or 11, **characterised in that** the vitamin is selected from a group comprising natural and synthetic compounds with a retinoid structure (vitamins A), vitamin B complex, ascorbic acids (vitamins C), calciferols (vitamins D), tocopherols (vitamins E), vitamins K, flavonoids and biotin.

13. Transport system as claimed in one of claims 10 to 12, **characterised in that** the at least one vitamin is selected from a group comprising retinol, retinyl acetate, retinyl palmitate, 3,4-didehydroretinol (vitamin A2), retinal, retinoic acid and provitamins such as α-, β-, γ-carotin, lutein, zeaxanthin, thiamine (vitamin B1) or thiamine hydrochloride or thiamine monomitrate, riboflavin (vitamin B2) or sodium-riboflavin-5-phoshate, niacin (vitamin B3) or nicotinic acid or neacin, pantothenic acid (vitamin B5) or calcium-D-pantothenate or sodium-D-pantothenate or D-panthenol, pyridoxine (vitamin B6) or pyridoxine hydrochloride or pyridoxine-5-phosphate or pyridoxine dipalmitate or pyridoxal phosphate, folic acid (vitamin B9) or pteroylglutamic acid, cobalamin (vitamin B 12) or cyanocobalamin or hydroxycobalamin, biotin, choline, inositol and p-aminobenzoic acid, L-ascorbic acid, sodium-L-ascorbate, calcium-L-ascorbate, potassium-L-ascorbate and L-ascorbyl-6-palmitate, ergocalciferol (vitamin D2), cholecalciferol (vitamin D3), 1,25-dihydroxycholecalciferol and the provitamins ergosterol or 7-dehydrocholesterol, D-α-tocopherol, DL-α-tocopherol, D-α-tocopheryl acetate, DL-α-tocopheryl acetate and D-α-tocopheryl acid succinate, phylloquinone (vitamin K1), menoquinone (vitamin K2), menadione (vitamin K3) and menadione hydroxyquinone (vitamin K4).

14. Transport system as claimed in claim 10 or 11, **characterised in that** the at least one mineral or the at least one trace element are selected on the basis of their importance to the organism, selected from a group comprising Na, K, Mg, Ca, Fe, I, Cu, Mn, Zn, Co, Mo, Se, Cr, F, Si, Ni, As, Sn, V, P, Cl, B, Al and Br.

15. Transport system as claimed in claim 10 or 11, **characterised in that** the at least one component is selected from a group comprising coenzyme Q-10, quercetin, bromelain, inositol, choline, pycnogenol, carnitine, taurine, mesoinosit.

16. Transport system as claimed in claim 10 or 11, **characterised in that** the at least one essential amino acid is selected from a group comprising histidine, isoleucine, leucine, lysin, methionine, phenylalanine, threonine, tryptophan, valine and arginine.

17. Transport system a claimed in claim 10 or 11, **characterised in that** the at least one fatty acid is selected from a group comprising linoleic acid, linolenic acid and arachidonic acid.

18. Method of transporting active substances, **characterised in that** the transport system used is as claimed in one of claims 1 to 17.

19. Transport system as claimed in one of claims 1 to 17 for use as a medicament.

20. Use of the transport system as claimed in claim 19 for manufacturing medicaments to treat nutritional deficiencies.

21. Use of the transport system as claimed in claim 20 for topical and oral application.

22. Use of the transport system as claimed in one of claims 1 to 17, **characterised in that** it is used in the pharmaceutical, cosmetic and food industry.

## Revendications

1. Système de transport pour des principes actifs comprenant des particules hybrides en au moins une couche de molécules de lipide et au moins un ligand lié par une unité entretoise, ligand qui est un peptide, **caractérisé en ce qu'**au moins un groupe polymérisable est incorporé dans la particule hybride.

2. Système de transport selon la revendication 1, **caractérisé en ce que** l'unité entretoise est formée par des acides aminés, une substance chimiquement inerte comme une nanoparticule, choisie parmi un groupe comprenant des nanotubes de carbone, des nanofilaments ou des colloïdes.

3. Système de transport selon la revendication 1 ou 2, **caractérisé en ce que** les molécules de lipide sont des lipides polymérisables et/ou des lipides « naturels » comme par exemple des stéroïdes, des glycolipides, des phospholipides, des sphingolipides, des polyisoprénoïdes.

4. Système de transport selon l'une quelconque des revendications précédentes, **caractérisé en ce que** le peptide est un oligopeptide.

5. Système de transport selon la revendication 4, **caractérisé en ce que** l'oligopeptide présente une longueur, choisie dans un intervalle avec une limite inférieure de 4 acides aminés, de préférence de 5 acides aminés, en particulier de 6 acides aminés et une limite supérieure de 18 acides aminés, de préférence de 20 acides aminés, en particulier de 22 acides aminés.

6. Système de transport selon la revendication 4 ou 5, **caractérisé en ce que** la séquence d'oligopeptides est complémentaire à la séquence d'un récepteur sur une cellule.

7. Système de transport selon l'une quelconque des revendications 4 à 6, **caractérisé en ce que** l'oligopeptide présente une séquence choisie parmi un groupe comprenant les séquences Gly-Arg-Gly-Asp-Ser-Pro (SEQ ID NO: 1), Tyr-Ile-Glu-Ser-Arg (SEQ ID NO: 2), et/ou Ala-Asp-Gly-Glu-Ala (SEQ ID NO: 3).

8. Système de transport selon l'une quelconque des revendications 4 à 6, **caractérisé en ce que** l'oligopeptide présente une séquence choisie parmi un groupe comprenant les séquences Val-Arg-Leu-Leu-Asn-Asn (SEQ ID NO: 4), Val-Arg-Leu Leu-Asn-Asn-Trp-Asp (SEQ ID NO: 5), Gly-Arg-Val-Arg-Leu-Leu-Asn-Asn (SEQ ID NO: 6), Met-Thr-Ala-Gly-Ala-Gly (SEQ ID NO: 7), Leu-Ser-Gly-Ala-Leu-Arg (SEQ ID NO: 8). Ile-Val-Ala-Ile-Leu-Ile-Cys-Ile-Leu-Ile-Leu-Leu-Thr-Met-Val-Leu-Leu-Phe-Val-Met -Trp-Met (SEQ ID NO: 9), Ile-Val-Ala-Ile-Leu-Ile-Cys-Ile-Leu-Ile-Leu-Leu (SEQ ID NO: 10), Ile-Val-Ala-Ile-Leu-Ile-Cys-Ile-Leu-Ile-Leu-Leu-Thr-Met-Val-Leu-Leu-Phe (SEQ ID NO: 11), Ile-Val-Ala-Ile-Leu-Ile (SEQ ID NO: 12), Cys-Ile-Leu-Ile-Leu-Leu (SEQ ID NO: 13), Thr-Met-Val-Leu-Leu-Phe (SEQ ID NO: 14) et/ou Leu-Phe-Val-Met-Trp-Met (SEQ ID NO: 15).

9. Système de transport selon l'une quelconque des revendications précédentes, **caractérisé en ce que** les particules hybrides forment des structures tridimensionnelles comme par exemple des vésicules, des microsphères, des nanoparticules, des tubes.

10. Système de transport selon l'une quelconque des revendications précédentes, **caractérisé en ce que** le principe actif est au moins un micronutriment.

11. Système de transport selon la revendication 10, **caractérisé en ce que** ledit au moins un micronutriment est au moins une substance choisie parmi le groupe comprenant des provitamines, des vitamines, des substances minérales et des oligoéléments, des acides aminés, des acides gras, des polyphénols, des hormones et des extraits d'organes, respectivement leurs produits de synthèse, comme par exemple la pancréatine, l'acide biliaire, la substance de base du cartilage.

12. Système de transport selon la revendication 10 ou 11, **caractérisé en ce que** la vitamine est choisie parmi le groupe comprenant des composés naturels et synthétiques à structure de rétinoïde (vitamine A), le complexe des vitamines B, l'acide ascorbique (vitamine C), les calciférols (vitamine D), les tocophérols (vitamine E), la vitamine K, les flavonoïdes et la biotine.

13. Système de transport selon l'une quelconque des revendications 10 à 12, **caractérisé en ce que** ladite au moins une vitamine est choisie parmi le groupe comprenant le rétinol, l'acétate de rétinyle, le palmitate de rétinyle, le 3,4-didéhydrorétinol (vitamine A2), le rétinal, l'acide rétinique et des provitamines, comme par exemple l'α-, le β-, le γ-carotène, la lutéine, la zéaxanthine, la thiamine (vitamine B1) respectivement le chlorhydrate de thiamine respectivement le mononitrate de thiamine, la riboflavine (vitamine B2) respectivement le sodium-riboflavine-5-phosphate, la niacine (vitamine B3) respectivement l'acide nicotinique respectivement le neacin, l'acide pantothénique (vitamine B5) respectivement le calcium-D-pantothénate respectivement le sodium-D-pantothénate respectivement le D-panthénol, la pyridoxine (vitamine B6) respectivement le chlorhydrate de pyridoxine respectivement le pyridoxine-5-phosphate respectivement le dipalmitate de pyridoxine respectivement le phosphate de pyridoxal, l'acide folique (vitamine B9) respectivement l'acide ptéroylglutamique, la cobalamine (vitamine B12) respectivement la cyanocobalamine respectivement l'hydroxycobalamine, la biotine, la choline, l'inosite et l'acide p-aminobenzoïque, l'acide L-ascorbique, le L-ascorbate de sodium, le L-ascorbate de calcium, le L-ascorbate de potassium et le L-ascorbyl-6-palmitate, l'ergocalciférol (vitamine D2), le cholécalciférol (vitamine D3), le 1,25-dihydroxycholécalciférol et les provitamines ergostérol respectivement 7-déhydrocholestérol, le D-α-tocophérol, le DL-α-tocophérol, l'acétate de D-α-tocophéryle, l'acétate de DL-α-tocophéryle et le succinate de D-α-tocophéryle, la phylloquinone (vitamine K1), la ménoquinone (vitamine K2), la ménadione (vitamine K3) et la ménadionehydroxyquinone (vitamine K4).

14. Système de transport selon la revendication 10 ou 11, **caractérisé en ce que** ladite au moins une substance minérale, respectivement ledit au moins un oligo-élément, dans l'ordre de leur signification pour l'organisme, sont choisis parmi un groupe comprenant Na, K, Mg, Ca, Fe, I, Cu, Mn, Zn, Co, Mo, Se, Cr, F, Si, Ni, As, Sn, V, P, Cl, B, Al et Br.

15. Système de transport selon la revendication 10 ou 11, **caractérisé en ce que** ledit au moins un composant est choisi parmi un groupe comprenant le coenzyme Q-10, la quercétine, la broméline, l'inositol, la choline, le pycnogénol, la carnitine, la taurine, le méso-inositol.

16. Système de transport selon la revendication 10 ou 11, **caractérisé en ce que** ledit au moins un acide aminé essentiel est choisi parmi le groupe comprenant l'histidine, l'isoleucine, la leucine, la lysine, la méthionine, la phénylalanine, la thréonine, le tryptophane, la valine et l'arginine.

17. Système de transport selon la revendication 10 ou 11, **caractérisé en ce que** ledit au moins un acide gras est choisi parmi le groupe comprenant l'acide linoléique, l'acide linolénique, l'acide arachidonique.

18. Procédé pour le transport de principes actifs, **caractérisé en ce que** l'on utilise le système de transport selon l'une quelconque des revendications 1 à 17.

19. Système de transport selon l'une quelconque des revendications 1 à 17 pour une utilisation comme médicament.

20. Utilisation du système de transport selon la revendication 19 à la préparation d'un médicament pour le traitement de carences alimentaires.

21. Utilisation du système de transport selon la revendication 20 pour une administration topique et orale.

22. Utilisation du système de transport selon l'une quelconque des revendications 1 à 17, **caractérisé en ce qu'**il est utilisé dans les industries pharmaceutiques, cosmétiques et alimentaires.
